# EUROPÄISCHE PATENTSCHRIFT

(11) **EP 0 653 930 B1**
(45) Veröffentlichungstag und Bekanntmachung des Hinweises auf die Patenterteilung: **11.06.1997**
(21) Anmeldenummer: 93915684.0
(22) Anmeldetag: 30.07.1993
(51) Int. Cl.: A61F 13/15

(54) **WEGWERFWINDEL**
DISPOSABLE NAPPY
COUCHE A JETER

(30) Priorität: 10.08.1992 DE 4226370
(43) Veröffentlichungstag der Anmeldung: 24.05.1995
(73) Patentinhaber: Paul Hartmann Aktiengesellschaft, D-89522 Heidenheim (DE)
(72) Erfinder: MALOWANIEC, Krzysztof, D., D-89522 Heidenheim (DE); JOHN, Astrid, D-89522 Heidenheim (DE)
(74) Vertreter: Becker, Maria, Dipl.-Phys.
(86) Internationale Anmeldenummer: DE9300683
(87) Internationale Veröffentlichungsnummer: WO9403136

(56) Entgegenhaltungen:
- US-A- 4 662 877
- US-A- 4 816 025
- US-A- 5 080 658

## Beschreibung

Die Erfindung betrifft eine Wegwerfwindel, wie sie im Oberbegriff des Patentanspruchs 1 beschrieben ist. Diese Wegwerfwindel weist auf der dem Körper zugewandten Seite eine hydrophobe, mit einer Öffnung versehene Vliesstoffauflage auf.

In der US-PS 4,662,877 ist eine Wegwerfwindel beschrieben, die eine Wäscheschutzfolie, eine flüssigkeitsdurchlässige Vliesstoffabdeckung, einen zwischen Vliesstoffabdeckung und Wäscheschutzfolie befindlichen rechteckigen Saugkörper und eine rechteckige hydrophobe Vliesstoffauflage aufweist. Die hydrophobe Vliesstoffauflage ist auf die flüssigkeitsdurchlässige Vliesstoffabdeckung, die kantenbündig mit der Wäscheschutzfolie abschliesst, aufgebracht. Wenn Flüssigkeit vom Saugkörper aufgenommen wird, wird auch die hydrophile Vliesstoffabdeckung befeuchtet. Inbesondere bei grösserem Flüssigkeitsanfall kann die Flüssigkeit zwischen hydrophober Vliesstoffauflage und Wäscheschutzfolie entlang des Vliesstoffmaterials der Vliesstoffabdeckung des Saugkörpers nach aussen zum Windelrand kriechen. Das Vliesstoffmaterial entwickelt dabei eine Dochtwirkung.

Es ist Aufgabe der Erfindung, die bekannte Wegwerfwindel derart zu verbessern, dass sie gegenüber Körperausscheidungen eine erhöhte Dichtigkeit aufweist.

Die Aufgabe wird erfindungsgemäss gelöst durch die kennzeichnenden Merkmale des Patentanspruchs 1. Diese Wegwerfwindel hat den Vorteil, dass sie zumindest im Schrittbereich eine optimale Dichtigkeit aufweist, da sich im Beinbereich der Windel kein hydrophiles Vliesstoffmaterial zwischen hydrophober Vliesstoffauflage und Wäscheschutzfolie befindet, durch die ein Flüssigkeitstransport nach aussen ermöglicht würde.

Die Ausgestaltung der Windel nach Anspruch 2 ermöglicht eine ebensolche Dichtigkeit auch in den anderen Windelbereichen und stellt sicher, dass die Haut eines Trägers der Windel nicht in Berührung mit der Wäscheschutzfolie kommt.

Mit der Ausgestaltung nach Anspruch 3 ist dieselbe Dichtigkeit in allen Windelrandbereichen gewährleistet. Dies ist vorteilhaft, wenn die die Windel tragende Person sich meistens in liegender Haltung befindet und so die Körperausscheidungen auch in die Taillenbereiche der Windel wandern.

Die Ausgestaltung nach Anspruch 4 vereinfacht das Aufbringen der flüssigkeitsdurchlässigen Vliesstoffabdeckung auf die Wäscheschutzfolie und den Saugkörper beim Herstellen der Windel.

Aufgrund von Elastifizierungsmitteln, die entlang der freien Ränder der sich in Längsrichtung der Windel erstreckenden Klappen angeordnet sind, stehen die Klappen beim Gebrauch der Windel vom Saugkörper ab. Durch die Säckchenbildung werden die Körperausscheidungen im Bereich des Saugkörpers gehalten und wandern nicht seitwärts in den Beinbereich.

Um einen unangenehmen Kontakt zwischen Haut und Elastifizierungsmittel zu vermeiden, sind diese innerhalb eines gefalteten freien Klappenrandteils angebracht.

Mit der Ausgestaltung der Windel nach Anspruch 7 sind die Elastifizierungsmittel über die stirnseitigen Öffnungsränder hinaus wirksam und bewirken somit ein Hochstehen der stirnseitigen zweiten Dichtklappen.

Im nachfolgenden wird die Erfindung anhand der Fig. 1 bis 3, die eine bevorzugte Ausgestaltung der Erfindung zeigen, im näheren beschrieben. Es zeigen:
- Fig. 1: eine Draufsicht auf eine flachgelegte Wegwerfwindel, bei der ein Teil weggebrochen ist;
- Fig. 2: einen Schnitt entlang der im Schrittbereich verlaufenden Linie II-II der Fig. 1, wobei die Wegwerfwindel in Gebrauchslage dargestellt ist;
- Fig. 3: einen Schnitt entlang der im Taillenbereich verlaufenden Linie III-III der Fig. 1.

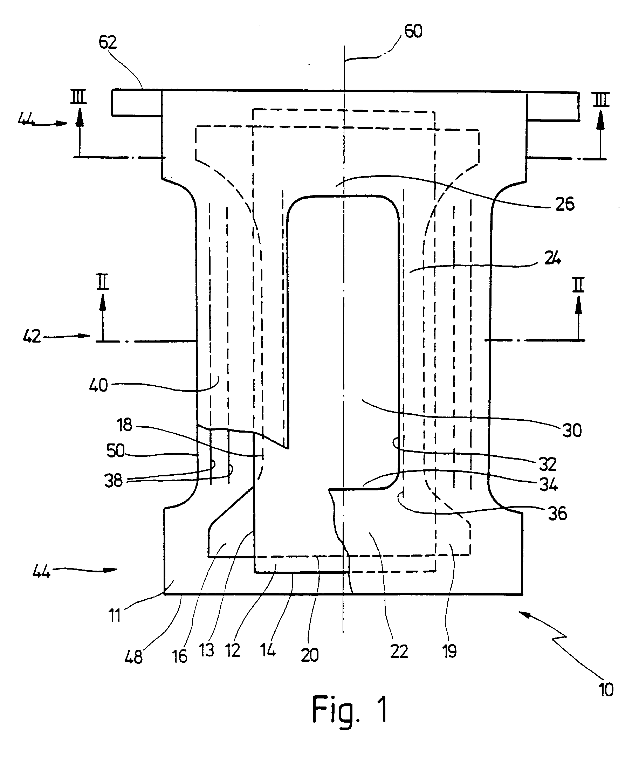

Fig. 1 zeigt eine bevorzugte Ausgestaltung einer erfindungsgemässen Wegwerfwindel 10 im flachgelegten Zustand mit gestreckter Lage der Elastifizierungsmittel.

Die Windel 10 ist bezüglich ihrer Längsachse 60 symmetrisch und teilt sich auf in zwei Taillenbereiche 44 und einen Schrittbereich 42. Die in den Taillenbereichen 44 symmetrisch zur Linie II-II liegenden Windelelemente sind jeweils nur einmal mit Bezugsziffern gekennzeichnet.

Die Windel 10 weist eine flüssigkeitsundurchlässige, sanduhrförmig zugeschnittene Wäscheschutzfolie 11 und einen darauf liegenden, ebenfalls sanduhrförmigen Saugkörper 16 auf. Der Saugkörper 16 ist mit seinen Längsseiten 18 und Stirnseiten 20 auf der Wäscheschutzfolie 11 durch eine flüssigkeitsdurchlässige Vliesstoffabdeckung 12 festgelegt. Diese weist einen rechteckigen Zuschnitt auf und überragt mit ihren Längsrändern 13 die Längsränder 18 des Saugkörpers 16 im Windelschrittbereich, während sie mit ihren Stirnenden 14 die Stirnenden 20 des Saugkörpers 16 teilweise überragt und mit ihren Rändern mit der Wäscheschutzfolie 11 verbunden ist mit Ausnahme im Bereich der vier Ecken 19 des Saugkörpers 16. Diese sind nicht von der Vliesstoffabdeckung 12 überdeckt. Die Längsränder 13 der Vliesstoffabdeckung 12 liegen im Abstand von den Seitenrändern 50 der Wäscheschutzfolie 11. Die Stirnenden 14 der Vliesstoffabdeckung 12 sind entweder kantenbündig mit einem Taillenrand 48 der Wäscheschutzfolie oder liegen, wie in Fig. 1 dargestellt, parallel zu den Seitenrändern innerhalb des Taillenrandes 48.

Die Wäscheschutzfolie 11, der Saugkörper 16 und die Vliesstoffabdeckung 12 werden von einer hydrophoben Vliesstoffauflage 22 überdeckt. Diese Vliesstoffauflage 22 hat die gleichen Aussenabmessungen wie die Wäscheschutzfolie 11, so dass sie kantenbündig mit dieser abschliesst. Die hydrophobe Vliesstoffauflage 22 kann mit dem Saugkörper 16 und der Wäscheschutzfolie 11 wenigstens an den Flächen verbunden sein, die von der hydrophilen Vliesstoffabdeckung 12 nicht abgedeckt sind. Die Vliesstoffauflage 22 kann im Windelschrittbereich 42 zusätzlich mit der Vliesstoffabdeckung 12 entlang ihrer Längsränder 13 ausserhalb der Längsseite 18 des Saugkörpers 16 verbunden sein. Dies gibt der Windel eine erhöhte Stabilität. Zusätzlich ist die Vliesstoffauflage 22 in den Taillenbereichen 44 zwischen den Stirnseiten 20 des Saugkörpers 16 und den Taillenrändern 48 der Wäscheschutzfolie 11 mit der Vliesstoffabdeckung 12 verbunden.

Zwischen Vliesstoffauflage 22 und Wäscheschutzfolie 11 befinden sich ausserhalb des Längsrandes 13 der Vliesstoffabdeckung 12 parallel zu den Seitenrändern 50 jeweils zwei elastische Bänder 38, die mit der Wäscheschutzfolie 11 und/oder der Vliesstoffauflage 22 verbunden sind. So sind im Windelschrittbereich 42 elastifizierte Seitenlappen 40 gebildet, die eine Abdichtung gegen die Beine eines Trägers bewirken.

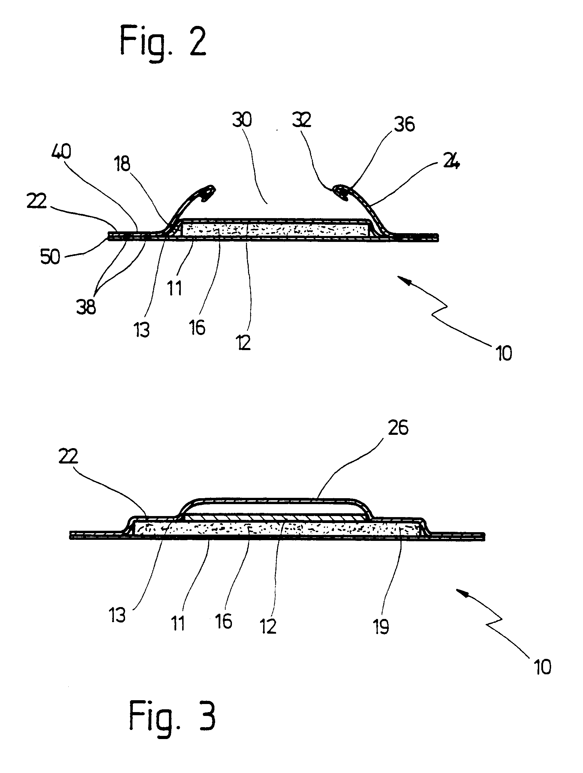

Die hydrophobe Vliesstoffauflage 22 weist eine zentrale Öffnung 30 auf, die durch freie Längsränder 32 und Stirnseiten 34 begrenzt ist. Entlang der Längsränder 32 sind Elastifizierungsmittel 36 vorgesehen, deren wirksame Strecke über die Stirnseiten 34 der Öffnung 30 hinausgeht. Die Elastifizierungsmittel 36 befinden sich auf der dem Saugkörper zugewandten Seite der Vliesstoffauflage 22 und werden durch ein Umklappen des Längsrandes 32 der Öffnung 30 umhüllt, wie in Fig. 2 dargestellt. Die Elastifizierungsmittel 36 können auch durch einen zusätzlichen Streifen Vlies abgedeckt oder in jeweils eine sich über die gesamte Länge der hydrophoben Vliesstoffauflage 22 erstreckende Falte eingebracht sein. Dadurch wird ein unangenehmer Kontakt zwischen Elastifizierungsmitteln 36 und der Haut verhindert.

In Gebrauchslage der Wegwerfwindel 10 kontrahieren die Elastifizierungsmittel 36 und bewirken damit ein Abstehen der Längsränder 32 der Öffnung 30 sowie hochragende Stirnseiten 34. Auf diese Weise sind erste Dichtklappen 24 entlang der Längsseite des Saugkörpers 16 (Fig. 2) sowie zweite Dichtklappen 26 (Fig. 3) an den Stirnseiten gebildet. Die Ränder 32 und 34 der Öffnung 30 wirken abdichtend gegen den Körper eines Trägers. Die Dichtklappen 24 und 26 halten die Körperausscheidungen im Bereich des Saugkörpers 16. Sie sind insbesondere dicht gegenüber flüssigen Körperausscheidungen, da sich zwischen der die Dichtklappen bildenden hydrophoben Vliesstoffauflage 22 und der Wäscheschutzfolie 11 kein flüssigkeitsdurchlässiges Vliesstoffmaterial befindet, durch das Flüssigkeit, insbesondere im Schrittbereich, nach aussen kriechen kann.

Die Öffnung 30 der Vliesstoffauflage 22 kann nicht nur eine abgerundete rechteckige Form, wie in Fig. 1 dargestellt, aufweisen, sondern es sind auch andere Formen möglich. So kann die Öffnung 30 beispielsweise auch eine ovale Form haben, wobei sich die längere Achse in Windellängsrichtung 60 erstreckt. Die Elastifizierungsmittel 36 sollten entlang der Längsseitenränder 32 verlaufen. Durch die ovale Form bekommen die ersten Dichtklappen 24 ausserhalb des Schrittbereiches 42 eine grössere Höhe. Dadurch kann die Aufnahmefähigkeit der Windel 10 erhöht werden. In einer anderen Ausgestaltungsform der hydrophoben Vliesstoffauflage 22 hat die Öffnung 30 eine trapezförmige Gestalt. Dadurch weisen die ersten Dichtklappen 24 eine von einem Taillenbereich zum anderen stetig zunehmende Höhe auf. In einer weiteren, ebenfalls nicht dargestellten Ausgestaltung ist die Öffnung 30 nicht zentral in der Vliesstoffauflage 22 positioniert. Sie kann beispielsweise in den vorderen oder rückwärtigen Taillenbereich 44 verschoben sein. Das bedeutet, dass die zweite Dichtklappe 26 in dem Taillenbereich 44, in den die Öffnung 30 verschoben wurde, eine niedrigere Höhe hat als im gegenüberliegenden Taillenbereich 44.

Dichtklappen 24 und 26 mit unterschiedlicher Höhe können also einfach durch eine speziell geformte Öffnung 30, die in die hydrophobe Vliesstoffauflage 22 eingebracht wird, hergestellt werden. Um ihre Dichtwirkung zu erzielen, müssen die Längsränder 32 der Öffnung 30 jeweils mit Elastifizierungsmitteln 36 versehen werden.

Gemäss einer weiteren, nicht dargestellten Ausgestaltung kann vorgesehen sein, dass sich entlang der Taillenränder 48 der Wäscheschutzfolie 11 elastische Elemente befinden, beispielsweise aus Schaumgummi bestehend. Elastische Elemente im Taillenbereich fördern das Abstehen der zweiten Dichtklappen 26 vom Saugkörper 16 und ergeben eine vorteilhaftere Passform der Wegwerfwindel 10. Vorteilhafterweise haben diese elastischen Elemente eine Breite, die sich vom Taillenrand 48 bis über die Stirnseite 20 des Saugkörpers 16 hinauserstreckt.

Die Herstellung der beschriebenen Wegwerfwindel 10 kann sich auf zwei Teile beziehen, nämlich zum einen auf die Wäscheschutzfolie 11 mit dem Saugkörper 16 und zum anderen auf das "Abdecksystem", das aus der flüssigkeitsdurchlässigen Vliesstoffabdeckung 12, der hydrophoben Vliesstoffauflage 22 und den Elastifizierungsmitteln 36, 38 besteht. Dabei kann das Herstellungsverfahren schnell umgeschaltet werden, wenn beispielsweise ein anderes "Abdecksystem" gewünscht wird.

Die Aufgliederung der Windel in Wäscheschutzfolie und Saugkörper einerseits und "Abdecksystem" andererseits bietet eine grössere Flexibilität in bezug auf Änderungen, die an der Windel oder dem Herstellungsverfahren vorgenommen werden.

## Patentansprüche

1. Wegwerfwindel (10) mit einer flüssigkeitsundurchlässigen Wäscheschutzfolie (11), einer mit dieser verbundenen, flüssigkeitsdurchlässigen Vliesstoffabdeckung (12) , einem zwischen Wäscheschutzfolie (11) und Vliesstoffabdeckung (12) befindlichen Saugkörper (16) mit zwei, sich jeweils entlang einer Längsseite (18) des Saugkörpers (16) erstreckenden, auf die Vliesstoffabdeckung (12) aufgebrachten ersten Dichtklappen (24), die jeweils ein sich längs erstreckendes, mit seinen Enden festgelegtes, bandförmiges Elastifizierungsmittel (36) aufweisen, und mit sich in den Endbereichen des Saugkörpers (16) quer zu den ersten Dichtklappen (24) erstreckenden zweiten Dichtklappen (26), wobei die Klappen (24, 26) den Rand (32, 34) einer im Windelschrittbereich (42) befindlichen Öffnung (30) einer hydrophoben, auf die flüssigkeitsdurchlässige Vliesstoffabdeckung (12) aufgebrachten Vliesstoffauflage (22) bilden,
**dadurch gekennzeichnet,**
dass die Breite der flüssigkeitsdurchlässigen Vliesstoffabdeckung (12) im Windelschrittbereich (42) kleiner ist als diejenige der Wäscheschutzfolie (11) und dass die hydrophobe Vliesstoffabdeckung (22) im Windelschrittbereich (42) die Längsränder (13) der flüssigkeitsdurchlässigen Vliesstoffabdeckung (12) überdeckt und mit der Wäscheschutzfolie (11) abdichtend verbunden ist.

2. Wegwerfwindel (10) nach Anspruch 1, dadurch gekennzeichnet, dass die hydrophobe Vliesstoffauflage (22) mit der Wäscheschutzfolie (11) im wesentlichen kantenbündig abschliesst.

3. Wegwerfwindel (10) nach Anspruch 1 oder 2, dadurch gekennzeichnet, dass die Längsabmessung der flüssigkeitsdurchlässigen Vliesstoffabdeckung (12) kleiner ist als diejenige der Wäscheschutzfolie (11).

4. Wegwerfwindel (10) nach einem der Ansprüche 1 und 2, dadurch gekennzeichnet, dass die flüssigkeitsdurchlässige Vliesstoffabdeckung (12) mit ihren Stirnenden (14) mit der Wäscheschutzfolie (11) im wesentlichen kantenbündig abschliesst.

5. Wegwerfwindel (10) nach einem der vorhergehenden Ansprüche, dadurch gekennzeichnet, dass die Elastifizierungsmittel (36) entlang der freien Ränder (32) der sich in Längsrichtung der Windel (10) erstreckenden ersten Klappen (24) angeordnet sind.

6. Wegwerfwindel (10) nach Anspruch 5, dadurch gekennzeichnet, dass die Elastifizierungsmittel (36) innerhalb des gefalteten freien Klappenrandteils (32) eingebracht sind.

7. Wegwerfwindel (10) nach einem der vorhergehenden Ansprüche, dadurch gekennzeichnet, dass die Enden der Elastifizierungsmittel (36) zwischen den stirnseitigen Öffnungsrändern (34) und den diesen benachbarten Rändern (48) der Wäscheschutzfolie (11) festgelegt sind.

## Claims

1. Disposable diaper (10) having a protective film (11) that is impermeable to liquids, a liquid-permeable non-woven fabric cover (12) connected with such film, an absorbent body (16) being arranged between the protective film (11) and the non-woven fabric cover (12) and comprising two first sealing flaps (24) extending respectively along the longitudinal edges (18) of the absorbent body (16) and applied on the non-woven fabric cover (12), each of the flaps comprising lengthwise extending strip-like elasticizing means (36) fixed on both ends, and having further second sealing sealing flaps (26) extending transversely relative to the first sealing flaps (24) in the end regions of the absorbent body (16), the flaps (24, 26) forming the edge (32, 34) of an opening (30) located in the crotch area (42) of the diaper in a hydrophobic non-woven fabric cover (22) applied on the liquid-permeable non-woven fabric cover (12),
**characterized in that**
the width of the liquid-permeable non-woven fabric cover (12) is smaller, in the crotch area of the diaper (42), than the width of the protective film (11) and that the hydrophobic non-woven fabric cover (22) overlies the longitudinal edges (13) of the liquid-permeable non-woven fabric cover (12) in the crotch area (42) of the diaper, and is connected in this area with the protective film (11) in sealing relationship.

2. Disposable diaper (10) according to claim 1, characterized in that the hydrophobic non-woven fabric layer (22) extends substantially flush with the edges of the protective film (11).

3. Disposable diaper (10) according to claim 1 or 2, characterized in that the longitudinal dimension of the liquid-permeable non-woven fabric cover (12) is smaller than that of the protective film (11).

4. Disposable diaper (10) according to any of claims 1 and 2, characterized in that the end faces (14) of the non-woven fabric cover (12) extend substantially flush with the edges of the protective film (11).

5. Disposable diaper (10) according to any of the preceding claims, characterized in that the elasticizing means (36) are arranged along the free edges (32) of the first flaps (24) that extend in the longitudinal direction of the diaper (10).

6. Disposable diaper (10) according to claim 5, characterized in that the elasticizing means (36) are arranged inside the folded free marginal portion of the flap (32).

7. Disposable diaper (10) according to any of the preceding claims, characterized in that the ends of the elasticizing means (36) are fixed between the edges of the opening (34) on the end faces and the respective adjacent edges (48) of the protective film (11).

## Revendications

1. Couche à jeter (10) avec une pellicule (11) imperméable pour protéger le linge, un recouvrement (12) en nappe de fibres perméable aux liquides relié à la pellicule, un corps absorbant (16), situé entre la pellicule de protection (11) du linge et le recouvrement (12) en nappe de fibres avec deux premiers abattants d'étanchement (24) s'étendant respectivement le long d'un côté longitudinal (18) du corps absorbant (16), fixés sur le recouvrement (12) en nappe de fibres et présentant un moyen d'élastification (36) en forme de bande allongée fixé avec ses extrémités, et un deuxième abattant d'étanchement (26) s'étendant dans les zones terminales du corps absorbant (16) transversalement aux premiers abattants d'étanchement (24), les abattants (24, 26) formant le bord (32, 34) d'une ouverture (30) d'une couche en nappe de fibre (22) hydrophobe fixée sur le recouvrement (12) en nappe de fibres et située dans la zone du pas (42) de la couche,
**caractérisée en ce que**
la largeur du recouvrement (12) en nappe de fibres perméable aux liquides dans la zone du pas de la couche (42) est inférieure à celle de la pellicule de protection (11) du linge et que le recouvrement hydrophobe (22) en nappe de fibres dans la zone du pas de la couche (42) recouvre les bords longitudinaux (13) du recouvrement (12) en nappe de fibres perméable aux liquides et est relié de façon étanche avec la pellicule (11) de protection du linge.

2. Couche à jeter (10) selon la revendication 1, caractérisée en ce que la couche hydrophobe (22) en nappe de fibres forme un bord quasi à fleur avec la pellicule (11) de protection du linge.

3. Couche à jeter (10) selon la revendication 1 ou 2, caractérisée en ce que la longueur du recouvrement (12) en nappe de fibres perméable aux liquides est inférieure à celle de la pellicule (11) de protection du linge.

4. Couche à jeter (10) selon l'une des revendications 1 et 2, caractérisée en ce que le recouvrement (12) en nappe de fibres perméable aux liquides forme avec ses extrémités frontales (14) un bord quasi à fleur avec la pellicule (11) de protection du linge.

5. Couche à jeter (10) selon l'une des revendications précédentes, caractérisée en ce que les moyens d'élastification (36) sont disposés le long des bords libres (32) des premiers abattants (24) s'étendant en direction longitudinale de la couche (10).

6. Couche à jeter (10) selon la revendication 5, caractérisé en ce que les moyens d'élastification (36) sont intégrés dans la partie libre pliée du bord de l'abattant (32).

7. Couche à jeter (10) selon l'une des revendications précédentes, caractérisée en ce que les extrémités des moyens d'élastification (36) sont prévues entre les bords d'ouverture (34) du côté frontale et les bords (48) contigus à ces derniers de la pellicule de protection du linge (11).
